# EUROPEAN PATENT APPLICATION

(11) **EP 4 778 463 A1**
(43) Date of publication of application: **22.07.2026**
(21) Application number: 24891794.0
(22) Date of filing: 15.11.2024
(51) Int. Cl.: A61B 5/346, A61B 5/00, A61B 5/339, G16H 10/60, G16H 40/20, G16H 40/60, G06F 21/62, G16H 80/00

(54) **METHOD, PROGRAM, AND APPARATUS FOR PROVIDING ELECTROCARDIOGRAM READING INFORMATION**

(30) Priority: 15.11.2023 KR 20230158309; 12.04.2024 KR 20240049675; 09.08.2024 KR 20240106970
(71) Applicant: Medical AI Co., Ltd., Seoul 06180 (KR)
(72) Inventor: KWON, Joonmyoung, Seoul 06180 (KR)
(74) Representative: Patentanwaltskanzlei Matschnig & Forsthuber OG
(86) International application number: PCT/KR2024/018024
(87) International publication number: WO 2025/105848

(57) **Abstract**

In accordance with an embodiment of the present disclosure, a method, a program, and a device for providing electrocardiogram reading information are provided. The method may comprise: acquiring a first user command through a user interface for an electrocardiogram reading service; and displaying, through the user interface, at least one of reading information regarding a disease or health status of a subject whose electrocardiogram is measured, or first reference information related to an artificial intelligence used for an electrocardiogram reading.

## Description

### TECHNICAL FIELD

The present disclosure relates to a data processing technology in the medical field, and more specifically, to a method for providing not only an electrocardiogram (hereinafter, "ECG") reading result based on artificial intelligence (hereinafter, "AI") but also additional information related to the AI used for an ECG reading.

### BACKGROUND OF THE INVENTION

An ECG test is a test that records the electrical activity of the heart. The ECG test is a relatively simple and cost-effective test method for checking the health status of the heart, and it plays an important role in the early diagnosis and management of heart disease. For example, an ECG signal measured through an ECG test may be used to check whether each part of the heart is functioning normally, whether the size and position of the heart are normal, and whether there is damage to the heart muscle. Furthermore, the ECG signal may be used to diagnose various heart-related problems and predict a person's health status based on such checks.

Meanwhile, as AI technology develops, attempts to analyze ECGs using AI technology are increasing. A service that predicts various heart diseases by inputting an ECG into a neural network-based model is a representative example. Such services generally provide merely the probability of disease occurrence as an analysis result based on the ECG. Therefore, it is difficult to externally verify the reliability of the reading result, and there is a possibility that doctors using the ECG reading service may not trust or use the reading result. Accordingly, for an AI's ECG reading result to be usefully employed in the actual medical field, it is important not only to provide the probability of disease occurrence as a reading result to the doctor who confirms the ECG reading result (i.e., the doctor who requested the ECG reading service) but also to provide additional information that can confirm the reliability of the reading result.

### SUMMARY OF THE INVENTION

### TECHNICAL PROBLEM

An object of the present disclosure is to provide a method for providing information regarding the specification and performance of an AI used for an ECG reading, in addition to the ECG reading result.

However, the problems to be solved by the present disclosure are not limited to the aforementioned problems, and other unmentioned problems can be clearly understood from the following description.

### TECHNICAL SOLUTION

In accordance with an embodiment of the present disclosure for achieving the objects as described above, a method for providing ECG reading information is provided. The method may comprise: acquiring a first user command through a user interface for an ECG reading service; and displaying, through the user interface, at least one of reading information regarding a disease or health status of a subject whose ECG is measured, or first reference information related to an AI used for an ECG reading.

Alternatively, the first reference information may comprise at least one of: information of a user who requested the ECG reading; cutoff information for a risk level provided as a reading result using the AI; information regarding data used for training the AI; or performance evaluation information of the AI.

Alternatively, the information of a user may comprise: information regarding a type of a user who measured an ECG of the subject whose ECG is measured, the information being recorded in ECG data of the subject whose ECG is measured.

Alternatively, the information regarding data used for training the AI may comprise at least one of: information on the number of training data or validation data; information on the number of patients recruited to generate the training data or the validation data; or information on the number of medical institutions that generated the training data or the validation data.

Alternatively, the performance evaluation information of the AI may comprise at least one of: information regarding a validation study for each medical institution where an ECG was measured; information regarding a performance metric according to the validation study for each medical institution; information regarding a validation study for each underlying condition of a subject whose ECG was measured; or information regarding a performance metric according to the validation study for each underlying condition.

Alternatively, the first reference information may vary depending on a type of a user who requested the ECG reading.

Alternatively, the first reference information may be generated by, when a type of a user recorded in ECG data of the subject whose ECG is measured is identified, processing pre-stored source information according to the identified type of the user.

Alternatively, the method may further comprise: acquiring a second user command through the user interface; and displaying, through the user interface, second reference information generated by adjusting the first reference information based on the second user command.

Alternatively, when the second user command comprises a first modification request for performance evaluation information of the AI included in the first reference information, cutoff information for a risk level provided as a reading result using the AI, which is included in the first reference information, may be dynamically changed based on the adjusted performance evaluation information according to the first modification request.

Alternatively, the first modification request may comprise a first adjustment value for at least one of a sensitivity or a specificity, which are performance metrics included in the performance evaluation information.

Alternatively, the cutoff information may be determined by searching for the first adjustment value on a Receiver Operating Characteristic (ROC) curve having the sensitivity and the specificity as variables.

Alternatively, when the second user command comprises a second modification request for cutoff information for a risk level provided as a reading result using the AI included in the first reference information, performance evaluation information of the AI, which is included in the first reference information, may be dynamically changed based on: ECG data input as a reading target according to the first user command; the reading information; and the adjusted cutoff information according to the second modification request.

Alternatively, when the second user command comprises text data, a modification request for the first reference information may be generated by inputting the second user command into a pre-trained large language model.

In accordance with an embodiment of the present disclosure for achieving the objects as described above, a computer program stored on a computer-readable storage medium is provided. The computer program, when executed by one or more processors, causes operations to be performed for providing ECG reading information. The operations may comprise: acquiring a first user command through a user interface for an ECG reading service; and displaying, through the user interface, at least one of reading information regarding a disease or health status of a subject whose ECG is measured, or first reference information related to an AI used for an ECG reading.

In accordance with an embodiment of the present disclosure for achieving the objects as described above, a computing device for providing ECG reading information is provided. The device may comprise: a processor including at least one core; a memory including program codes executable by the processor; and an input/output unit for providing a user interface that displays at least one of reading information regarding a disease or health status of a subject whose ECG is measured, or first reference information related to an AI used for an ECG reading.

### ADVANTAGEOUS EFFECTS

The present disclosure not only provides an ECG reading result but also provides additional information that can explain the specification and performance of the AI used for the reading, thereby increasing the reliability of the reading result and helping a customer to accurately understand the reading result.

### BRIEF DESCRIPTION OF THE DRAWINGS

FIG. 1 is a block diagram of a computing device according to an embodiment of the present disclosure.
FIG. 2 is a block diagram of a system according to an embodiment of the present disclosure.
FIG. 3 is a sequence diagram illustrating operations by a system according to an embodiment of the present disclosure.
FIG. 4 is a conceptual diagram illustrating a relation between performance evaluation information and cutoff information according to an embodiment of the present disclosure.
FIG. 5 is a flowchart illustrating a method for providing ECG reading information according to an embodiment of the present disclosure.
FIG. 6 is a flowchart illustrating a method for providing ECG reading information according to another embodiment of the present disclosure.

### DETAILED DESCRIPTION OF THE EMBODIMENTS

Hereinafter, embodiments of the present disclosure will be described in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and should not be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" should be understood to mean any of the natural inclusive permutations. For example, if "X uses A or B" is used in the present disclosure, unless otherwise specified or clear from the context, it may be interpreted as any one of the cases where X uses A, X uses B, or X uses both A and B.

The term "and/or" as used in the present disclosure should be understood to refer to and include all possible combinations of one or more of the associated listed concepts.

The terms "comprise" and/or "comprising" as used in the present disclosure should be understood to mean the presence of a specific feature and/or component. However, the terms "comprise" and/or "comprising" should be understood as not precluding the presence or addition of one or more other features, other components, and/or combinations thereof.

In the present disclosure, unless otherwise specified or clear from the context to indicate a singular form, the singular generally should be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data through a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented through software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within a scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have a problem-solving ability by optimizing a parameter that connects a node or a neuron through learning. A neural network "model" may include a single neural network or a set of neural networks in which a plurality of neural networks are combined.

The term "control graphic" as used in the present disclosure may be understood as a graphic object that generates a visible result by executing a command when a user interface is manipulated. In other words, a "control graphic" may be understood as a basic unit constituting a user interface, which displays content provided to a user or a user interface element that a user can manipulate.

The above description of terms is for the purpose of facilitating the understanding of the present disclosure. Therefore, it should be noted that if the above terms are not explicitly stated as limiting the content of the present disclosure, they are not used in a sense that limits the technical spirit of the content of the present disclosure.

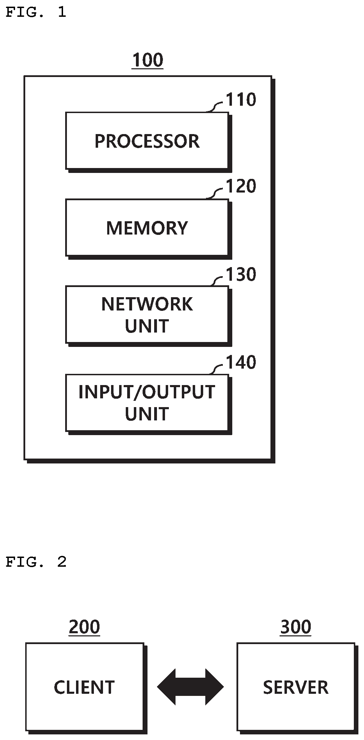

FIG. 1 is a block diagram of a computing device according to an embodiment of the present disclosure.

A computing device (100) according to an embodiment of the present disclosure may be a hardware device or a part of a hardware device that performs comprehensive processing and calculation of data, or it may be a software-based computing environment connected via a communication network. For example, the computing device (100) may be a server, which is a subject that performs intensive data processing functions and shares resources, or it may be a client that shares resources through interaction with a server. In addition, the computing device (100) may be a cloud system in which a plurality of servers and clients interact to process data comprehensively. The above description is only one example related to the type of the computing device (100), and therefore, the type of the computing device (100) may be variously configured within a scope understandable by a person of ordinary skill in the art based on the content of the present disclosure.

Referring to FIG. 1, the computing device (100) according to an embodiment of the present disclosure may comprise a processor (110), a memory (120), a network unit (130), and an input/output unit (140). However, FIG. 1 is only an example, and the computing device (100) may include other components to implement a computing environment. In addition, only some of the disclosed components may be included in the computing device (100).

The processor (110) according to an embodiment of the present disclosure may be understood as a component unit including hardware and/or software for performing computing operations. For example, the processor (110) may process commands generated as a result of user interaction through a user interface. In addition, the processor (110) may perform data processing for machine learning by reading a computer program. The processor (110) may process operational processes such as processing of input data for machine learning, feature extraction for machine learning, and error calculation based on backpropagation. The processor (110) for performing such data processing and operations may include a central processing unit (CPU), a general-purpose graphics processing unit (GPGPU), a tensor processing unit (TPU), an application-specific integrated circuit (ASIC), or a field-programmable gate array (FPGA). The types of the processor (110) described above are only examples, and the type of the processor (110) may be variously configured within a scope understandable by a person of ordinary skill in the art based on the content of the present disclosure.

The processor (110) may generate a user interface for an ECG reading service. The ECG reading service may be understood as a service that performs an ECG reading and provides an ECG reading result upon a request from an ECG reading requester. The user interface for the ECG reading service may include a control graphic for acquiring a user command, such as a control graphic for receiving an ECG reading request and a control graphic for receiving ECG data. In addition, the user interface for the ECG reading service may include a control graphic for providing information related to the ECG reading, such as reading information regarding a disease or health status of a subject whose ECG is measured, and reference information related to an AI that performed the ECG reading. When the user interface is implemented through the input/output unit (140), which will be described later, the aforementioned control graphics may be arranged and visually expressed in specific areas of the user interface.

Meanwhile, the processor (110) may perform an ECG reading using AI. The processor (110) may generate ECG reading data by inputting ECG data into an AI. In this case, the ECG reading data may include reading information regarding a disease or health status of a subject whose ECG is measured. The reading information may include information that can be used for diagnosing a user's disease or health status, such as the type of disease, the probability of disease occurrence, and the probability of aggravation of a disease or health status. For example, when a user command requesting an ECG reading is acquired through the user interface, the processor (110) may input the ECG data input through the user command into a neural network model. In this case, the neural network model may be a pre-trained model that outputs a probability regarding the occurrence of a disease or the aggravation of a health status based on features extracted from the ECG data. In this case, the training may be supervised learning performed based on labeled data, and depending on the structure of the neural network model, it may be unsupervised learning or self-supervised learning. The processor (110) may generate reading data by performing an analysis on the ECG data through the neural network model. The processor (110) may express the reading data generated through the neural network model as a control graphic of the user interface.

A memory (120) according to an embodiment of the present disclosure may be understood as a constituent unit including hardware and/or software for storing and managing data processed in a computing device (100). That is, the memory (120) may store any form of data generated or determined by the processor (110) and any form of data received by the network unit (130). For example, the memory (120) may include at least one storage medium of a flash memory type, a hard disk type, a multimedia card micro type, a card type memory, a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. In addition, the memory (120) may include a database system that controls and manages data in a predetermined structure. The foregoing types of the memory (120) are only one example, and the type of the memory (120) may be variously configured within a scope understandable by a person of ordinary skill in the art based on the content of the present disclosure.

The memory (120) may manage data, a combination of data, and program codes executable by the processor (110), which are necessary for the processor (110) to perform operations, in a structured and organized manner. For example, the memory (120) may store medical data received through the network unit (130), which will be described later. The memory (120) may store a program code that stores rules for processing medical data, a program code that operates a neural network model to perform learning by receiving medical data as input, a program code that operates a neural network model to perform inference according to the purpose of the computing device (100) by receiving medical data as input, and processed data generated as the program code is executed.

A network unit (130) according to an embodiment of the present disclosure may be understood as a component unit that transmits and receives data through any form of known wired and wireless communication system. For example, the network unit (130) may perform data transmission and reception using a wired or wireless communication system such as a local area network (LAN), wideband code division multiple access (WCDMA), long term evolution (LTE), wireless broadband internet (WiBro), 5th generation mobile communication (5G), ultra wide-band, ZigBee, radio frequency (RF) communication, wireless LAN, wireless fidelity (Wi-Fi), near field communication (NFC), or Bluetooth. The aforementioned communication systems are only examples, and therefore, the wired and wireless communication system for data transmission and reception of the network unit (130) may be variously applied in addition to the examples described above.

The network unit (130) may receive data necessary for the processor (110) to perform operations through wired/wireless communication with any system or any client. In addition, the network unit (130) may transmit data generated through the operations of the processor (110) through wired/wireless communication with any system or any client. For example, the network unit (130) may receive biosignal data through communication with a database in a hospital environment, a cloud server that performs tasks such as standardizing medical data, a client such as a smart watch, or a medical computing device. The network unit (130) may transmit output data of a neural network model, and intermediate data and processed data derived from the operational process of the processor (110), through communication with the aforementioned database, server, client, or computing device.

The input/output unit (140) according to an embodiment of the present disclosure may be understood as a component unit including hardware and/or software for implementing a user interface. That is, the input/output unit (140) may visualize and output any form of data generated or determined by the processor (110) and any form of data received by the network unit (130). In addition, the input/output unit (140) may receive a user input that generates a command to be delivered to the processor (110), an arbitrary system connected to the computing device (100) by wired/wireless communication, or an arbitrary client. For example, the input/output unit (140) may include a display module that can implement a touch screen or output visualized information, such as a liquid crystal display (LCD), a thin film transistor-liquid crystal display (TFT-LCD), an organic light-emitting diode (OLED), a flexible display, and a 3D display. In addition, the input/output unit (140) may include an input module that can recognize a user's motion, voice, or other action, such as a camera, a microphone, a keyboard, and a mouse. The modules described above are only examples, and the modules included in the input/output unit (140) may be variously configured within a scope understandable by a person of ordinary skill in the art based on the content of the present disclosure, in addition to the examples described above.

The input/output unit (140) may implement a user interface to output graphics generated through the processor (110) or receive a user command generated by a user operation and deliver it to the processor (110). For example, the input/output unit (140) may output control graphics for receiving a user command related to an ECG reading and control graphics for providing information in a specific area of the user interface. In addition, the input/output unit (140) may receive a user command for the control graphics output in a specific area of the user interface. In this case, the user command for a specific graphic may be understood as an input signal generated by a user operation on the specific graphic. Also, the user operation on a specific graphic may mean an action that a user can perform through the input/output unit (140), such as a click, a double-click, a hover, a flick, a pinch, and a spread on the specific graphic. The input/output unit (140) may cause operations to be performed for providing a user interface based on user control by receiving a user command and delivering it to the processor (110).

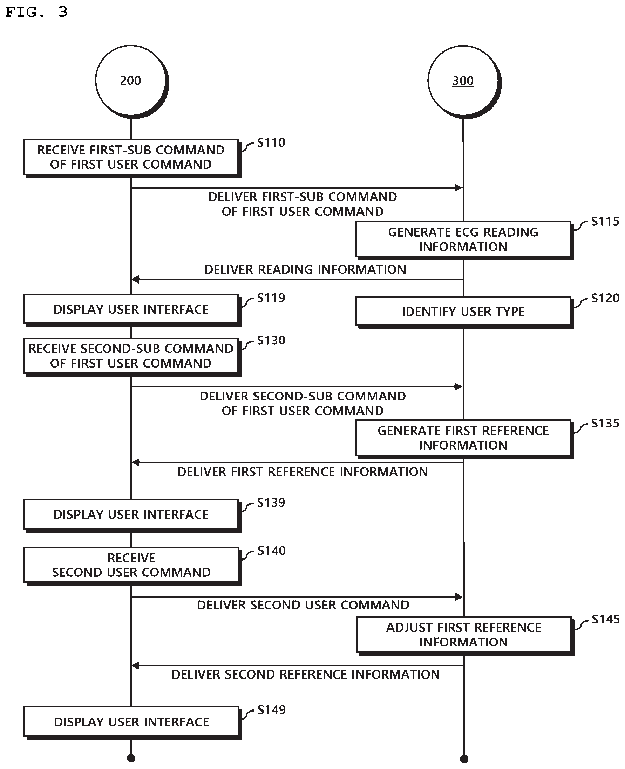

FIG. 2 is a block diagram of a system according to an embodiment of the present disclosure. FIG. 3 is a sequence diagram illustrating operations by a system according to an embodiment of the present disclosure.

Referring to FIG. 2, a system according to an embodiment of the present disclosure may include a client (200) that provides a user interface to a user, and a server (300) that processes information acquired through the user interface or information provided to the user interface. For example, the client (200) may correspond to the computing device (100) of FIG. 1. The client (200) may control graphics implemented in a user interface using at least one of a command or data acquired through interaction with a user. The client (200) may deliver at least one of a user command or data acquired through the user interface to the server (300). The server (300) may correspond to the form of the computing device (100) of FIG. 1, excluding the input/output unit (140). The server (300) may perform processing on information to be provided through the user interface, using at least one of a command or data acquired through the user interface. Specifically, the server (300) may generate visualized information to be provided to the user through the user interface, using an AI model. The server (300) may deliver the processed information to the client (200), and the client (200) may provide the processed information to the user through the user interface.

Referring to FIG. 3, the client (200) may acquire a first sub-command of a first user command through a user interface for an ECG reading service (S110). In this case, the first sub-command of the first user command may include a user's ECG reading request and ECG data which is a reading target. The client (200) may deliver the first sub-command of the first user command to the server (300). The server (300) may generate ECG reading information based on the ECG data included in the first sub-command of the first user command (S115). Specifically, the server (300) may input the ECG data included in the first sub-command of the first user command into a neural network model. The server (300) may extract ECG features from the ECG data through the neural network model. In this case, the ECG features relate to an ECG waveform and may include a P wave, a QRS complex, an R peak, and the like. The server (300) may derive a numerical value indicating a probability of disease occurrence or aggravation, or a probability of health status aggravation, based on the ECG features through the neural network model. The server (300) may generate reading information regarding a disease or health status of a subject whose ECG is measured by comparing the numerical value derived as an output of the neural network model with a cutoff. For example, if the numerical value derived as the output of the neural network model is less than the cutoff, the server (300) may determine that a risk of occurrence of a specific disease or a risk of aggravation of a health status is low, and may classify the subject whose ECG is measured into a low-risk group. If the numerical value derived as the output of the neural network model is equal to or greater than the cutoff, the server (300) may determine that a risk of occurrence of a specific disease or a risk of aggravation of a health status is high, and may classify the subject whose ECG is measured into a high-risk group. When the ECG reading information is generated, the server (300) may deliver the ECG reading information to the client (200). The client (200) may display the ECG reading information through the user interface (S119).

The server (300) may identify a type of a user who requested an ECG reading based on ECG data included in the first sub-command of the first user command (S120). For example, the server (300) may check information regarding the type of the user recorded in the ECG data included in the first sub-command of the first user command. Since the ECG data included in the first sub-command of the first user command is in an XML format, the server (300) may determine the type of the user who requested the ECG reading by identifying text regarding the type of the user recorded in the XML-formatted ECG data. In this case, the type of the user may be classified into a general hospital, a primary hospital, or a health screening center according to a type of a medical institution. In addition, the general hospital may be further classified into an outpatient clinic, an emergency room, or an intensive care unit. The server (300) may identify the type of the user who requested the ECG reading in this manner, and may provide additional information necessary for the user in addition to the ECG reading result. Meanwhile, although step S120 is expressed as being performed separately from step S115 in FIG. 3, step S120 may be performed simultaneously with step S115.

The client (200) may acquire a second sub-command of the first user command through the user interface (S130). In this case, the second sub-command of the first user command may include a request for reference information regarding the AI used for the ECG reading. Although step S130 is expressed as being performed separately from step S110 in FIG. 3, step S130 may also be performed simultaneously with step S110. The reference information may include information regarding a specification or performance of the AI used for the ECG reading. The client (200) may deliver the second sub-command of the first user command to the server (300). The server (300) may generate first reference information related to the AI used for the ECG reading based on the second sub-command of the first user command (S135). When the type of the user is identified through step S120 from the ECG data of the subject whose ECG is measured, the server (300) may process pre-stored source information according to the type of the user. The source information may be understood as a set of all information used for training and inference of the AI. Specifically, the source information may include ECG information, information of the subject whose ECG is measured, information of the medical institution where the ECG was measured, information of the underlying condition of the subject whose ECG is measured, clinical study information related to the ECG reading, or training information and evaluation information of the AI used for the reading service. The server (300) may determine the first reference information by collecting and processing information related to the type of the user from this source information. For example, a user working in an emergency room may have a need to "see only clear myocardial infarctions, even if some myocardial infarctions are missed." In contrast, a user at a health screening center may have a need to "lower the cutoff criterion to avoid missing any patients, even if it means misdiagnosing a non-myocardial infarction patient as a myocardial infarction patient." Thus, for an emergency room user, it may be necessary to raise the cutoff for reading, while for a health screening center user, it may be necessary to lower the cutoff for reading. As such, because the content of the required information may vary depending on the type of the user, the server (300) may extract and process information from the source information based on the type of the user to generate first reference information tailored to the type of the user. Specifically, the server (300) may extract ECG data and reading information from the source information based on the type of the user. The server (300) may calculate a performance metric by applying a cutoff determined according to the type of the user, based on the ECG data and reading information extracted from the source information. The server (300) may generate the first reference information by aggregating the extracted and calculated information.

Meanwhile, the first reference information may include at least one of: information of a user who requested the ECG reading; cutoff information for a risk level provided as a reading result using the AI; information regarding data used for training the AI; or performance evaluation information of the AI. The cutoff information for the risk level provided as the reading result using the AI may be understood as information corresponding to a criterion for judging a disease or health status. A cutoff value included in the cutoff information may be a pre-set value based on a clinically acceptable performance evaluation metric of the AI, or it may be a value that is dynamically changed according to a type of a user or a user's request. The information regarding data used for training the AI may include at least one of: information on the number of training data or validation data; information on the number of patients recruited to generate the training data or the validation data; or information on the number of medical institutions that generated the training data or the validation data. The performance evaluation information of the AI may include at least one of: information regarding a validation study for each medical institution where an ECG was measured; information regarding a performance metric according to the validation study for each medical institution; information regarding a validation study for each underlying condition of a subject whose ECG was measured; or information regarding a performance metric according to the validation study for each underlying condition. Here, the performance metric may include at least one of an accuracy, a sensitivity, or a specificity for an output of the AI. In addition, the subject whose underlying condition was validated may include at least one of a pregnant woman, a patient with a left bundle branch block (LBBB) condition, a patient with an atrial fibrillation with rapid ventricular response (RVR) condition, or a patient complaining of chest pain.

When such first reference information is generated, the server (300) may deliver the first reference information to the client (200). The client (200) may display the first reference information through the user interface (S139). The client (200) and the server (300) may increase the reliability of the AI reading result by processing and providing the first reference information.

The client (200) may acquire a second user command through the user interface (S140). In this case, the second user command may include a modification request to adjust some or all of the detailed contents included in the first reference information. The client (200) may deliver the second user command to the server (300). The server (300) may adjust the first reference information based on the second user command to generate second reference information (S145). For example, even if the server (300) identifies the type of the user and provides reference information tailored to the type of the user, from the user's perspective, it may be necessary to adjust the reference information in detail or to have new reference information that suits the user's own needs. Therefore, the server (300) may transform the first reference information based on the second user command, which represents the user's needs, to generate reference information that reflects the user's needs. When a user inputs a second user command requesting to adjust the sensitivity and specificity, which are performance metrics of the AI, to values desired by the user, the server (300) may redetermine a cutoff value by searching for the adjustment values included in the second user command on a Receiver Operating Characteristic (ROC) curve, for which sensitivity and specificity are variables. The server (300) may generate second reference information by modifying the performance evaluation information included in the first reference information based on the adjustment values included in the second user command and the re-determined cutoff value. When a user inputs a second user command requesting to adjust the cutoff, which is a reading criterion, to a value desired by the user, the server (300) may re-calculate performance metrics by re-analyzing the ECG data and its reading information based on the cutoff. In this case, the re-analysis may be an operation of re-comparing the output value of the AI included in the reading information with the cutoff input through the second user command. The re-analysis may be an operation of filtering performance metrics based on the cutoff input through the second user command from source information where performance metrics for each cutoff are organized. The server (300) may generate second reference information by modifying the cutoff information and performance evaluation information included in the first reference information.

Meanwhile, the second user command may be numerical data regarding the information included in the first reference information, or it may be text data expressing a user request. For example, a user may input a second user command in text form, such as "I want to see performance metrics of the AI reading for only patients with LBBB as an underlying condition," through the user interface. If the second user command is such text data, the server (300) may input the second user command into a pre-trained large language model to parse a meaning embedded in the text and adjust the reference information. In addition, the server (300) may generate a modification request for the first reference information through the large language model to which the second user command is input. That is, if the second user command is in text form, the server (300) may use a large language model to clearly recognize the user's command.

When such second reference information is generated, the server (300) may deliver the second reference information to the client (200). The client (200) may display the second reference information through the user interface (S149). That is, the client (200) and the server (300) may provide reference information that reflects the user's needs, in addition to the reference information suitable for the type of the user, by generating the second reference information.

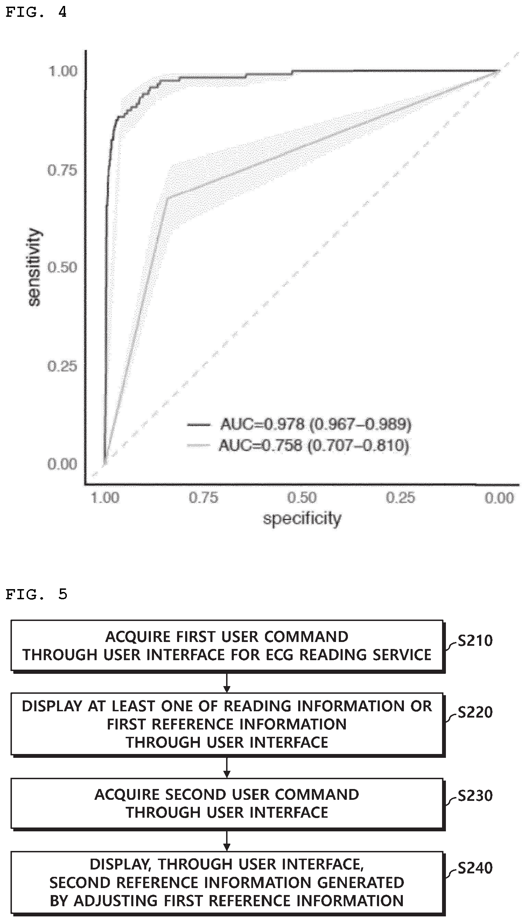

FIG. 4 is a conceptual diagram illustrating a relation between performance evaluation information and cutoff information according to an embodiment of the present disclosure.

The performance evaluation information included in the reference information according to an embodiment of the present disclosure may include performance metrics such as sensitivity and specificity. Sensitivity and specificity are metrics that indicate the performance of a test itself and are unique values for the specific test. For example, if a test for a specific disease has a sensitivity of 90% and a specificity of 95%, these values remain constant. Regardless of test results, the sensitivity and specificity do not change. Sensitivity is the proportion of individuals who are correctly identified as positive by the test among those actually having the disease. That is, it indicates how effectively the test identifies people with the disease. Specificity is the proportion of individuals who are correctly identified as negative by the test among those actually not having the disease. That is, it indicates how effectively the test distinguishes healthy people. Therefore, sensitivity and specificity are values determined by the design and performance of the test.

In an emergency room, patients with chest pain are classified into three risk levels for acute myocardial infarction. The highest risk group is defined as a "rule-in" group, the lowest risk group as a "rule-out" group, and the intermediate-risk group as an "observation" group for clinical treatment. In this case, the user considers the following to be clinically acceptable levels: a performance of at least 99% sensitivity and 99.5% negative predictive value (NPV) for determining "rule-out" for the low-risk group; and a performance of 90% specificity and 60-80% positive predictive value (PPV) for determining "rule-in" for the high-risk group.

A user may set a desired sensitivity and specificity through a user interface for an ECG reading service. When the sensitivity and specificity set by the user are input through the user interface, a corresponding cutoff may be determined through an ROC curve. An ROC curve represents sensitivity and 1-specificity (false positive rate) for various cutoffs. Each point on the ROC curve corresponds to a specific cutoff. Therefore, a cutoff may be searched for on the ROC curve based on the sensitivity and specificity set by the user. The determined cutoff may be reflected in the reference information provided to the user.

FIG. 5 is a flowchart illustrating a method for providing ECG reading information according to an embodiment of the present disclosure.

Referring to FIG. 5, a client (200) according to an embodiment of the present disclosure may acquire a first user command through a user interface for an ECG reading service (S210). The first user command may include a first sub-command of the first user command, which includes a user's ECG reading request and ECG data that is a reading target, and a second sub-command of the first user command, which includes a request for reference information regarding an AI used for the ECG reading. The client (200) may implement a user interface through an input/output unit to provide a user with control graphics for generating the first user command. The client (200) may generate the first user command through a user input operation on the control graphics of the user interface.

The client (200) may display, through the user interface, at least one of reading information regarding a disease or health status of a subject whose ECG is measured, or first reference information related to an AI used for the ECG reading (S220). The first reference information may include at least one of: information of a user who requested the ECG reading; cutoff information for a risk level provided as a reading result using the AI; information regarding data used for training the AI; or performance evaluation information of the AI. The first reference information may be information that varies depending on a type of a user who requested the ECG reading. When the first user command is acquired (S120), the client (200) may recognize user information recorded in the ECG data included in the first user command, and generate the first reference information according to the recognized information. Specifically, when a type of a user recorded in the ECG data of the subject whose ECG is measured is identified, the first reference information may be generated by processing pre-stored source information according to the identified type of the user. In this case, the processing may be an operation of extracting necessary information from the source information, or an operation of re-combining or generating information from the source information through a pre-trained neural network model.

The client (200) may acquire a second user command through the user interface for an ECG reading service (S230). The second user command may include a modification request to adjust some or all of the detailed contents included in the first reference information. The client (200) may implement a user interface through an input/output unit to provide a user with control graphics for generating the second user command. In this case, the control graphics for generating the second user command may be the same as the control graphics for generating the first user command. The client (200) may generate the second user command through a user input operation on the control graphics of the user interface. Meanwhile, the second user command may be included in the first user command. Therefore, step S240, which will be described later, may be performed sequentially after step S210 is performed, even if step S230 is not performed. That is, step S230 may be omitted.

The client (200) may display, through the user interface, second reference information generated by adjusting the first reference information based on the second user command (S240). When the second user command includes a first modification request for performance evaluation information of the AI included in the first reference information, the cutoff information for the risk level provided as the reading result using the AI, included in the first reference information, may be dynamically changed based on the adjusted performance evaluation information according to the first modification request. The first modification request includes a first adjustment value for at least one of a sensitivity or a specificity, which are performance metrics included in the performance evaluation information. The cutoff information is determined by searching for the first adjustment value on an ROC curve having the sensitivity and the specificity as variables. When the second user command includes a second modification request for the cutoff information for the risk level provided as the reading result using the AI, included in the first reference information, the performance evaluation information of the AI, which is included in the first reference information, is dynamically changed based on: the ECG data input as a reading target according to the first user command; the reading information; and the adjusted cutoff information according to the second modification request.

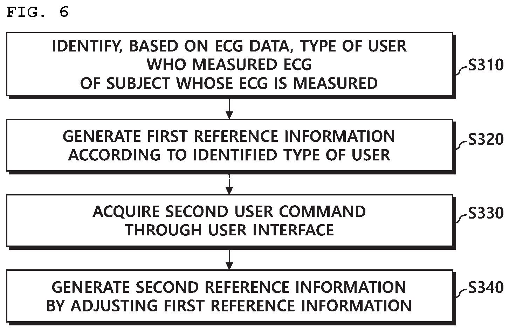

FIG. 6 is a flowchart illustrating a method for providing ECG reading information according to another embodiment of the present disclosure.

Referring to FIG. 6, a server (300) according to an embodiment of the present disclosure may identify a type of a user who measured an ECG of a subject whose ECG is measured, based on ECG data (S310). When a user command requesting an ECG reading is acquired, the server (300) may check the type of the user recorded in the ECG data. In this case, the type of the user may be classified according to a type of a medical institution. For example, the type of the user may be classified into a general hospital, a primary hospital, a health screening center, and the like. The general hospital may be further classified into an outpatient clinic, an emergency room, or an intensive care unit.

The server (300) may generate first reference information according to the type of the user identified through step S310 (S320). The reference information required may differ depending on the type of the user, such as the cutoff, which is the ECG reading criterion, and the performance metrics that may be derived therefrom. Therefore, the server (300) may generate the first reference information by processing pre-stored source information tailored to the type of the user. The source information may include training data information, validation data information, clinical study information, and the like used in the construction of the AI. This source information may be periodically updated.

The server (300) may acquire a second user command through a user interface for an ECG reading service (S330). When the second user command is received through the user interface implemented via the client (200), the server (300) may acquire the second user command through communication with the client (200). Meanwhile, as in step S230, the second user command may be included in a first user command requesting an ECG reading. Therefore, step S340, which will be described later, may be performed sequentially even if step S320 is not performed. That is, step S330 may be omitted.

The server (300) may adjust the first reference information to generate second reference information (S340). Since the detailed information included in the first reference information affects the determination of each piece of information, the server (300) may perform the adjustment of the detailed information included in the first reference information considering such effects. For example, if a user wants to change the cutoff, which is the reading criterion, the server (300) may modify not only the cutoff information included in the first reference information but also the reading information and performance evaluation information that are affected by the change in the cutoff information. Through this operation of the server (300), a user may easily check other reference information that changes accordingly while changing the desired reference information.

The various embodiments of the present disclosure described above may be combined with additional embodiments, and may be modified within a scope understandable by a person of ordinary skill in the art the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts should be interpreted as being included in the scope of the present disclosure.

## Claims

1. A method for providing electrocardiogram reading information, the method comprising:
acquiring a first user command through a user interface for an electrocardiogram reading service; and
displaying, through the user interface, at least one of reading information regarding a disease or health status of a subject whose electrocardiogram is measured, or first reference information related to an artificial intelligence used for an electrocardiogram reading.

2. The method of claim 1, wherein the first reference information comprises at least one of:
information of a user who requested the electrocardiogram reading;
cutoff information for a risk level provided as a reading result using the artificial intelligence;
information regarding data used for training the artificial intelligence; or
performance evaluation information of the artificial intelligence.

3. The method of claim 2, wherein the information of a user comprises:
information regarding a type of a user who measured an electrocardiogram of the subject whose electrocardiogram is measured,
the information being recorded in electrocardiogram data of the subject whose electrocardiogram is measured.

4. The method of claim 2, wherein the information regarding data used for training the artificial intelligence comprises at least one of:
information on the number of training data or validation data;
information on the number of patients recruited to generate the training data or the validation data; or
information on the number of medical institutions that generated the training data or the validation data.

5. The method of claim 2, wherein the performance evaluation information of the artificial intelligence comprises at least one of:
information regarding a validation study for each medical institution where an electrocardiogram was measured;
information regarding a performance metric according to the validation study for each medical institution;
information regarding a validation study for each underlying condition of a subject whose electrocardiogram was measured; or
information regarding a performance metric according to the validation study for each underlying condition.

6. The method of claim 1, wherein the first reference information varies depending on a type of a user who requested the electrocardiogram reading.

7. The method of claim 6, wherein the first reference information is generated by, when a type of a user recorded in electrocardiogram data of the subject whose electrocardiogram is measured is identified,
processing pre-stored source information according to the identified type of the user.

8. The method of claim 1, further comprising:
acquiring a second user command through the user interface;
and
displaying, through the user interface, second reference information generated by adjusting the first reference information based on the second user command.

9. The method of claim 8, wherein, when the second user command comprises a first modification request for performance evaluation information of the artificial intelligence included in the first reference information,
cutoff information for a risk level provided as a reading result using the artificial intelligence, which is included in the first reference information,
is dynamically changed based on the adjusted performance evaluation information according to the first modification request.

10. The method of claim 9, wherein the first modification request comprises a first adjustment value for at least one of a sensitivity or a specificity, which are performance metrics included in the performance evaluation information.

11. The method of claim 10, wherein the cutoff information is determined by searching for the first adjustment value on a Receiver Operating Characteristic (ROC) curve having the sensitivity and the specificity as variables.

12. The method of claim 8, wherein, when the second user command comprises a second modification request for cutoff information for a risk level provided as a reading result using the artificial intelligence included in the first reference information,
performance evaluation information of the artificial intelligence, which is included in the first reference information, is dynamically changed based on:
electrocardiogram data input as a reading target according to the first user command;
the reading information; and
the adjusted cutoff information according to the second modification request.

13. The method of claim 8, wherein, when the second user command comprises text data,
a modification request for the first reference information is generated by inputting the second user command into a pre-trained large language model.

14. A computer program stored on a computer-readable storage medium, the computer program, when executed by one or more processors, causing operations to be performed for providing electrocardiogram reading information,
the operations comprising:
acquiring a first user command through a user interface for an electrocardiogram reading service; and
displaying, through the user interface, at least one of reading information regarding a disease or health status of a subject whose electrocardiogram is measured, or first reference information related to an artificial intelligence used for an electrocardiogram reading.

15. A computing device for providing electrocardiogram reading information, the device comprising:
a processor including at least one core;
a memory including program codes executable by the processor; and
an input/output unit for providing a user interface that displays at least one of reading information regarding a disease or health status of a subject whose electrocardiogram is measured, or first reference information related to an artificial intelligence used for an electrocardiogram reading.
